# EUROPÄISCHE PATENTSCHRIFT

(11) **EP 2 317 949 B1**
(45) Veröffentlichungstag und Bekanntmachung des Hinweises auf die Patenterteilung: **14.06.2017**
(21) Anmeldenummer: 09775603.5
(22) Anmeldetag: 20.08.2009
(51) Int. Cl.: A61B 17/86

(54) **KNOCHENSCHRAUBENSET**
BONE SCREW SET
ENSEMBLE DE VIS À OS

(30) Priorität: 20.08.2008 AT 12932008
(43) Veröffentlichungstag der Anmeldung: 11.05.2011
(73) Patentinhaber: Medizinische Universität Wien, 1090 Wien (AT)
(72) Erfinder: ERHART, Jochen, A-3400 Klosterneuburg (AT); UNGER, Ewald, A-1210 Wien (AT); MAYR, Winfried, A-2340 Mödling (AT)
(74) Vertreter: Rentsch Partner AG
(86) Internationale Anmeldenummer: PCT/AT2009/000321
(87) Internationale Veröffentlichungsnummer: WO 2010/019979

(56) Entgegenhaltungen:
- EP-A- 1 825 826
- WO-A-00/38586
- WO-A-94/16636
- WO-A-2007/109302
- WO-A-2009/044395
- FR-A- 2 655 840
- US-A1- 2005 107 791
- US-A1- 2008 188 896

## Beschreibung

Die Erfindung betrifft ein Knochenschraubenset zur Fixierung von zwei Knochenfragmenten, insbesondere zur Behandlung einer Kahnbeinfraktur oder einer Kahnbeinpseudoarthrose, umfassend eine erste Knochenschraube und wenigstens eine zweite Knochenschraube oder einen fixierbaren Stift als Verdrehschutz bei Torsionsbelastung der zu verbindenden Knochenfragmente. Weiters wird ein Verfahren zur Fusion von zwei Knochenfragmenten offenbart, insbesondere zur Behandlung einer Kahnbeinfraktur oder einer Kahnbeinpseudoarthrose, unter Verwendung eines derartigen Knochenschraubensets.

Die Behandlung von Knochenfrakturen verlangt häufig eine interne Fixierung der Knochenfragmente in der gewünschten Position. Zu diesem Zweck wurde bereits eine Vielzahl von Fixierungsmitteln vorgeschlagen, welche u.a. Marknägel, Bohrdrähte, Platten und Schrauben umfassen.

Die Schrauben, welche zur Fixierung von Knochenfragmenten verwendet werden, sind häufig als Kompressionsschrauben ausgebildet. Ein solche kompressionsschraube ist beispielsweise aus WO 94/16636 A1 bekannt. Falls eine komplette Versenkung der Kompressionsschraube in den Knochen erforderlich ist, muss das Implantat in unterschiedlichen axialen Teilbereichen Gewinde mit unterschiedlichen Ganghöhen aufweisen. Wenn die unterschiedlichen Gewindebereiche in die miteinander zu verbindenden Knochenfragmente eingreifen, wird durch ein weiteres Einschrauben der Kompressionsschraube eine Relativbewegung der Knochenfragmente zueinander und folglich eine Kompression der Knochenfragmente aufeinander bewirkt. Nachteilig bei isolierter Verwendung dieser Kompressionsschrauben ist jedoch, dass nur eine Fixierung der Knochenfragmente in Achsrichtung der eingesetzten Schraube möglich ist und die Knochenfragmente gegen Rotation um die Schraubenachse nicht gesichert sind. Es sind zwar Fixierungsmittel bekannt, die zusätzlich eine Fixierung um die Rotationsachse des Implantats durch Querbohrungen und durch diese Querbohrungen geführte Splinten ermöglichen, jedoch lässt sich eine solche Sicherung bei kleineren Knochen und unter gewissen anatomischen Gegebenheiten nicht ohne weiteres anwenden. Speziell bei der Kahnbeinfraktur ist eine minimalinvasive Operation mit nur einem, möglichst klein zu haltenden Zugang wünschenswert. Eine Dissektion der Weichteile, welche erforderlich wäre, um geführte Quersplinte in Querbohrungen einzusetzen, ist nicht zuletzt wegen der Kompromittierung der spezifischen Durchblutung des Kahnbeines zu vermeiden. Eine Dissektion von Kapsel und Bändern des Handgelenkes mit entsprechend postoperativen Nachteilen wäre ebenso Voraussetzung für diese Art der Stabilisierung. Insbesondere bei Pseudoarthrosen (Falschgelenkbildungen) des Kahnbeines müssten die Splinte (Querverbolzungen) über oft nur sehr kleine, gelenktragende Anteile des Kahnbeines gesetzt werden, welche zudem als wichtige Lastüberträger fungieren müssen.

WO 2007/109302 A2 zeigt eine Vorrichtung zur Behandlung von Knochenfrakturen, beispielsweise von Oberschenkelhalsfrakturen. Eine Ankerschraube ist in einem vorgebohrten Loch angeordnet und ist mittels einem Gewinde mit dem Oberschenkelkopf-Fragment verbunden. Am proximalen Ende des Oberschenkelknochens ist ein Knochennagel oder eine Knochenplatte angebracht. Eine zweite Schraube verläuft ebenfalls in einem vorgebohrten Kanal in der Knochenstruktur und in einem Kanal der Ankerschraube, und weist ein Gewinde auf, das mit einem Gewinde im Kanal der Ankerschraube wechselwirkt. Der Kopf der zweiten Schraube ist auf einer Schulter einer Öffnung in Nagel abgestützt. Wird die Schraube eingeschraubt, so verschiebt sie sich im gebohrten Kanal in medialer Richtung zum Oberschenkelkopf, bis der Schraubenkopf auf dem Nagel aufsteht. Wird nun weitergedreht, so wird nun stattdessen die Ankerschraube mit dem daran fixierten Oberschenkelkopf-Fragment in Richtung Nagel gezogen, und die Bruchflächen werden zusammengepresst. In einer gezeigten Ausführungsform sind die Schrauben so ausgebildet, dass der Umfang der Ankerschraube sich in eingesetztem Zustand mit dem Umfang der zweiten Schraube teilweise überschneidet, so dass die zweite Schraube teilweise im Umfang der Ankerschraube steckt.

Eine rotationsstabile Zugschraubenosteosynthese über ein und denselben Zugang ist überall dort wünschenswert, wo keine offene Dissektion aus anatomischen Gründen möglich ist oder die kleine Dimension des Fragmentes die Verwendung eines zweiten, unabhängigen Implantates nicht zulässt. Dies ist neben der Kahnbeinfraktur und der Kahnbeinpseudoarthrose beispielsweise bei der Stabilisierung von kleinen gelenktragenden Fragmenten, bei der Fusion von Handwurzelknochen oder Fingergliedern oder aber auch bei der Stabilisierung der Densfraktur des zweiten Halswirbels der Fall.

Die vorliegende Erfindung zielt nun darauf ab, zusätzlich zur axialen Fixierung eine ausreichende Rotationsstabilität um die Achse des Implantats auch bei der Behandlung von Frakturen kleinerer Knochen zu gewährleisten, wobei die oben genannten Probleme umgangen werden sollen.

Zur Lösung dieser Aufgabe ist ein Knochenschraubenset der eingangs genannten Art erfindungsgemäß dahingehend weitergebildet, dass die erste Knochenschraube als Kompressionsschraube ausgebildet ist und die erste und die zweite Knochenschraube miteinander zusammenwirkende Eingriffsmittel aufweisen, um die zweite Knochenschraube in einer mit wenigstens einem Teilbereich derselben aus dem Querschnitt der ersten Knochenschraube heraustretenden Position relativ zur ersten Knochenschraube zu fixieren. Dadurch, dass die erste und die zweite Knochenschraube miteinander zusammenwirkende Eingriffsmittel aufweisen, gelingt es, die relative Position der beiden Knochenschrauben zueinander durch das Zusammenwirken der beiden Schrauben zu fixieren, sodass die Fixierungskräfte nicht vom umliegenden Knochenmaterial aufgebracht werden müssen. Dadurch, dass die zweite Knochenschraube in einer mit wenigstens einem Teilbereich derselben aus dem Querschnitt der ersten Knochenschraube heraustretenden Position relativ zur ersten Knochenschraube fixiert werden kann, kann der resultierende Gesamtquerschnitt des Implantats, bestehend aus einer ersten und einer zweiten Schraube, nach dem Einsetzen der ersten Knochenschraube so gewählt werden, dass der Querschnitt des eingesetzten Implantats ohne das Setzen von Querbohrungen asymmetrisch bzw. nicht rotationssymmetrisch ist, sodass ein Verdrehen der beiden Knochenfragmente zueinander unterbunden wird.

Mit Vorteil ist das Knochenschraubenset hierbei derart weitergebildet, dass die miteinander zusammenwirkenden Eingriffsmittel von einem Gewinde der zweiten Knochenschraube und einem mit diesem in Eingriff bringbaren Gewinde der ersten Knochenschraube gebildet sind. Bei einer solchen Ausführung könnte nach dem Setzen der ersten Knochenschraube allenfalls ein dünner Kanal entlang der äußeren Hüllkurve der ersten Knochenschraube in den Knochen gebohrt werden, in welchen eine beispielsweise selbstschneidende zweite Schraube eingesetzt wird, welche entlang der ersten Schraube in den Knochen eingeschraubt werden kann, um auf diese Weise eine Rotation der Knochenfragmente zueinander zu verhindern. Bevorzugt wäre eine selbstschneidende zweite Schraube, die sich ihren Weg beim Eindrehen selbst bahnt und dabei eine fixierende radiale Spreizkraft entwickelt. Dabei sind die erste und die zweite Knochenschraube mit Vorteil in einer im Wesentlichen parallel zueinander angeordneten Position zueinander fixierbar.

Gemäß der vorliegenden Erfindung ist das Knochenschraubenset dahingehend weitergebildet, dass die erste Knochenschraube an ihrem Außenumfang eine Längsnut aufweist, die zumindest über einen Teil ihrer Länge mit einem Innengewinde versehen ist. Bei einer solchen Ausbildung kann die zweite Knochenschraube mit ihrem Außengewinde in das in die Längsnut der ersten Schraube eingedrehte Gewinde der erste Knochenschraube eingreifen, sodass nach dem Einsetzen der beiden Schrauben ein aus zwei einander überlappenden Kreisen zusammengesetzter Gesamtquerschnitt des Implantats resultiert, wodurch eine Rotation der Knochenfragmente verhindert wird. Die Längsnut an der ersten Knochenschraube erstreckt sich hierbei beispielsweise von der Wurzel der Schraube in Richtung der Spitze der Schraube. Da die erste Schraube vollständig intraossär zu liegen kommt und deren Wurzel damit für den Operateur nicht sichtbar ist, ist eine Führungshilfe für das Bohren und Setzen der zweiten Schraube über ein und denselben Zugang notwendig. Diese kann an der ersten Schraube oder am Schraubenzieher der ersten Schraube fixiert werden.

Die Erfindung ist hierbei mit Vorteil dahingehend weitergebildet, dass die Tiefe der Längsnut in Richtung zur Spitze der ersten Knochenschraube abnimmt, wodurch es gelingt, den Querschnitt der zweiten Knochenschraube in unterschiedlichen axialen Bereichen des Implantats unterschiedlich stark aus dem gedachten kreisförmigen Querschnitt der ersten Knochenschraube, der resultieren würde, wenn die Gesamtheit aus Längsnut und zweiter Schraube nicht vorgesehen wäre, heraustreten zu lassen.

Wenn die Tiefe der Längsnut durch Ansteigen der Gewindeprofilhöhe in Richtung zur Spitze der ersten Knochenschraube abnimmt und zusätzlich der Kerndurchmesser der zweiten Knochenschraube zur Wurzel hin zunimmt, entfernt sich die Längsachse der zweiten Knochenschraube beim Eindrehen derselben und insbesondere gegen Ende des Einschraubvorgangs von der Längsachse der ersten Knochenschraube, wodurch eine Spreizkraft aufgebracht wird.

Das Knochenschraubenset kann bevorzugt auch so weitergebildet sein, dass das Gewinde der zweiten Schraube sowohl an der Spitze als auch an der Wurzel abgeflacht ist, um bei den letzten Umdrehungen durch das Vorbringen der zweiten Schraube in Längsrichtung zusätzlich eine Entfernung der Querschnitte der ersten und zweiten Schraube zu erwirken. Dadurch würde die zweite Schraube aus dem kreisförmigen Querschnitt der ersten Schraube verstärkt bei den letzten Umdrehungen heraustreten und das umliegende Knochengewebe komprimieren. Dadurch würde das Knochenschraubenset eine festere Verankerung mit dem umliegenden Knochengewebe bilden, um wiederum eine erhöhte Rotationsstabilität für die Knochenfragmente zu erreichen.

Bei einer alternativen Ausführungsform kann das erfindungsgemäße Knochenschraubenset dahingehend weitergebildet sein, dass die erste Knochenschraube eine Durchbrechung für die Aufnahme der zweiten Knochenschraube aufweist, deren Achse einen spitzen Winkel, insbesondere 10 bis 40°, mit der Schraubenachse der ersten Knochenschraube einschließt. Bei einer solchen Anordnung wird eine besonders starke Sicherung gegen Verdrehen erreicht, wobei die zweite Schraube die erste vollständig durchsetzt und sich insgesamt ein X-förmiger Gesamtquerschnitt des eingesetzten Implantats ergibt. Die erfindungsgemäße Vorrichtung kann hierbei mit Vorteil dahingehend weitergebildet sein, dass die Durchbrechung ein Innengewinde für das Einschrauben der zweiten Knochenschraube aufweist, wodurch wiederum das umliegende Knochenmaterial von den Kräften zur relativen Fixierung der beiden Schrauben zueinander freigehalten wird.

In bevorzugter Weise ist das erfindungsgemäße Knochenschraubenset dahingehend weitergebildet, dass der Durchmesser der ersten Knochenschraube größer ist als der Durchmesser der zweiten Knochenschraube, vorzugsweise das 1,5 - 2,5-fache des Durchmessers der zweiten Knochenschraube beträgt.

Mit Vorteil ist das erfindungsgemäße Knochenschraubenset dahingehend weitergebildet, dass die erste Knochenschraube konisch ausgebildet ist. Durch das Setzen einer konischen Bohrung in den miteinander zu verbindenden Knochenfragmenten und das darauf folgende Einsetzen einer konischen Schraube und insbesondere einer Kompressionsschraube wird erreicht, dass der Bereich der Spitze der Schraube mit dem proximalen Knochenfragment beim Einsetzen der Schraube nicht in Eingriff gelangt, was insbesondere dann, wenn, wie bei Kompressionsschrauben üblich, selbstschneidende Gewinde zum Einsatz gelangen, zu einer nachteiligen Veränderung der Bohrungsfläche im Knochen für den nachfolgenden, eigentlich für diesen Bereich gedachten Gewindeteil führen würde.

Mit Vorteil ist das erfindungsgemäße Knochenschraubenset dahingehend weitergebildet, dass die Gewindesteigung des Außengewindes der ersten Knochenschraube zur Erzielung einer Kompression im Bereich der Schraubenwurzel kleiner gewählt ist als im Bereich der Schraubenspitze, was dazu führt, dass gegen Ende des Eindrehens der Kompressionsschraube in die reponierten Knochenfragmente das proximale Knochenfragment auf das distale Knochenfragment gepresst wird, was für eine Beschleunigung des Heilungsprozesses allgemein als wünschenswert angesehen wird.

Die Erfindung kann bevorzugt dahingehend weitergebildet sein, dass die erste und die zweite Knochenschraube ein selbstschneidendes Außengewinde aufweisen, wodurch eine Bohrung ausreicht, um die Kompressionsschraube zu führen und in den Knochenfragmenten zu verankern. Das Schneiden eines separaten Gewindes mit Hilfe eines entsprechenden zusätzlichen Werkzeugs ist in diesem Fall nicht von Nöten.

Gemäß einer bevorzugten Ausführungsform der vorliegenden Erfindung ist das Knochenschraubenset dahingehend weitergebildet, dass die zweite Knochenschraube einen gewindelosen Bereich an der Wurzel aufweist. Nach einer nicht erfindungsgemässen Ausführungsform ist die zweite Knochenschraube als gewindeloser Einschubbolzen (Splint) ausgebildet ist, der z.B. hohl und mit geschlitzten Enden ausgeführt ist und durch Einführen eines passenden Stiftes nach der Positionierung verkeilbar ist.

Gemäß einer bevorzugten Ausführungsform der vorliegenden Erfindung ist das Knochenschraubenset dahingehend weitergebildet, dass die erste Knochenschraube geschlitzt ausgeführt ist und ausdrückbare verdrehschützende Elemente aufweist. Die verdrehschützenden Elemente werden dabei insbesondere durch das Einführen der zweiten Schraube oder eines Stiftes herausgedrückt.

Mit Vorteil weist das Knochenschraubenset gemäß der vorliegenden Erfindung eine Mehrzahl von miteinander zusammenwirkenden Knochenschrauben auf, wobei die erste und weitere Knochenschrauben miteinander zusammenwirkende Eingriffsmittel aufweisen, um die weiteren Knochenschrauben in einer mit wenigstens einem Teilbereich der selben aus dem Querschnitt der ersten Knochenschraube heraustretenden Position relativ zur ersten Knochenschraube zu fixieren. Mit einem solchen Set lassen sich mehrere Schrauben relativ zur ersten Schraube anordnen, sodass es möglich wird, den Querschnitt des gesamten Implantats weiter zu verändern, um eine weitere Sicherung der Knochenfragmente gegen Rotation zu gewährleisten.

Das Verfahren zur Fusion von zwei Knochenfragmenten, insbesondere zur Behandlung einer Kahnbeinfraktur, unter Verwendung eines Knochenschraubensets, umfassend eine erste Knochenschraube und eine zweite Knochenschraube, wobei die erste Knochenschraube als Kompressionsschraube ausgebildet ist und die erste und die zweite Knochenschraube miteinander zusammenwirkende Eingriffsmittel aufweisen, umfasst die folgenden Schritte:
- Reponieren der Knochenfragmente,
- Setzen eines Zielbohrdrahtes
- Bohren über den Zielbohrdraht durch die beiden miteinander zu verbindenden Knochenfragmente,
- Einschrauben der ersten Knochenschraube in die zwei Knochenfragmente, um die Bruchflächen der Knochenfragmente in der reponierten Stellung aneinander zu drücken,
- Einbringen der zweiten Knochenschraube in die Knochenfragmente entlang einer von der Achse der ersten Knochenschraube verschiedenen Achse, wobei die zweite Knochenschraube in eine Ausnehmung der ersten Knochenschraube zu liegen kommt, und Ineingriffbringen der Eingriffsmittel der ersten und der zweiten Knochenschraube, um die zweite Knochenschraube in einer mit wenigstens einem Teilbereich derselben aus dem Querschnitt der ersten Knochenschraube heraustretenden Position relativ zur ersten Knochenschraube zu fixieren.

Das Reponieren der Knochenfragmente und das Setzen der Bohrung erfolgt nach bekannten Methoden. Im Allgemeinen erfolgt das Setzen der Bohrung mit einem Bohrdraht, auf welchen die erste Knochenschraube, die eine zentrale Bohrung aufweist, aufgeschoben ist, sodass sie nachdem die Bohrung ausgeführt wurde, gleitend zum Ansatz der Bohrung geführt und in den Knochen eingeschraubt werden kann. Die erste Knochenschraube wird hierbei soweit eingebracht, um den gewünschten Kompressionseffekt zu erzielen, woraufhin die zweite Knochenschraube in die Ausnehmung der ersten Knochenschraube eingebracht wird, sodass die Eingriffsmittel der ersten und der zweiten Knochenschraube in Eingriff gelangen und die zweite Knochenschraube entlang der ersten Knochenschraube in den Knochen eindringt oder aus der ersten Knochenschraube austretend in den Knochen eindringt. Wenn die zweite Knochenschraube in ihre Zielposition gebracht wurde, weist das gesamte Implantat einen Querschnitt zur Längsachse auf, der nicht rotationssymmetrisch ist, sodass ein Verdrehen der beiden Knochenfragmente zueinander verhindert wird.

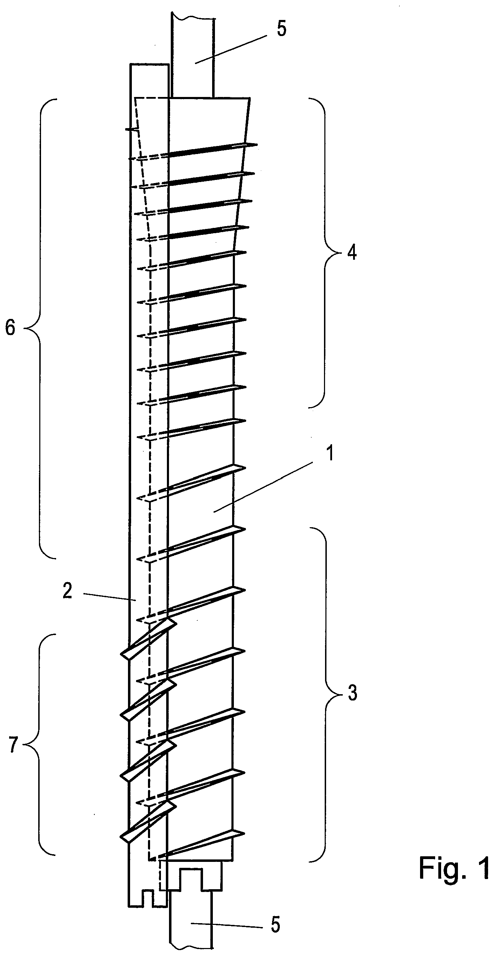

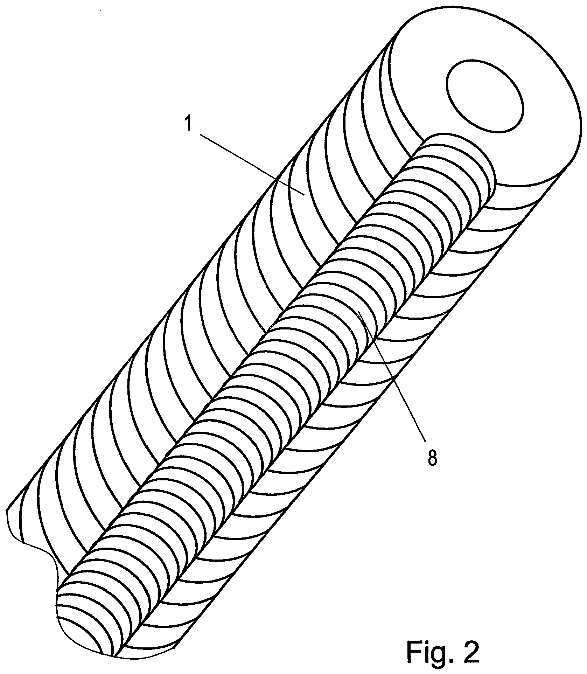

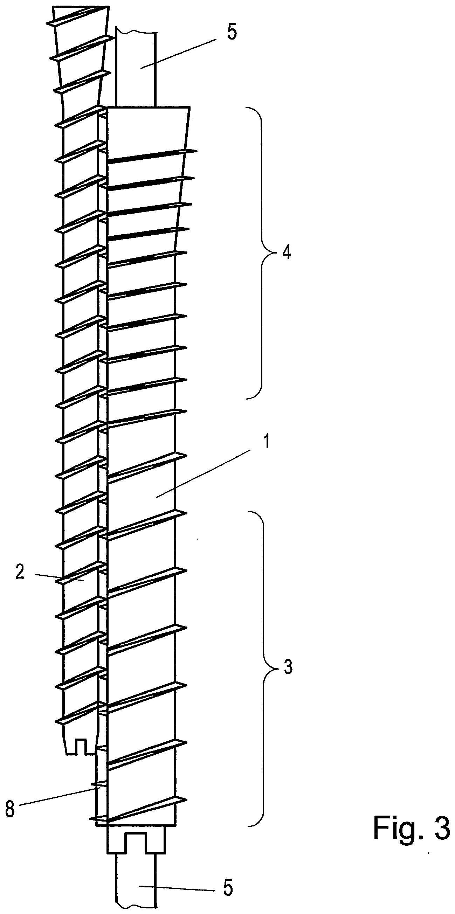

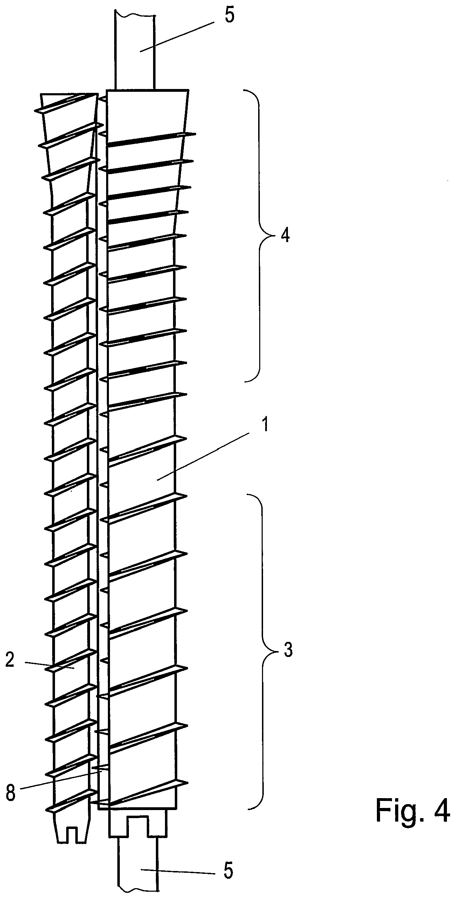

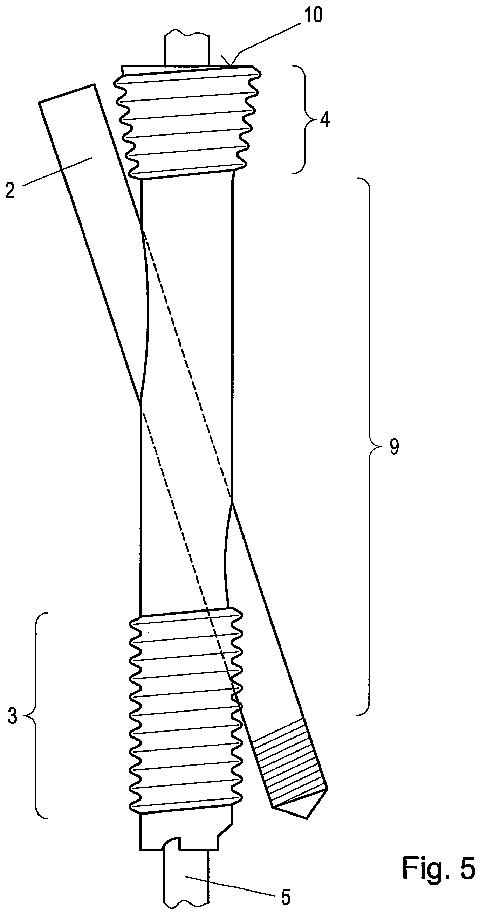

Die Erfindung wird nachfolgend anhand von in der Zeichnung schematisch dargestellten Ausführungsbeispielen näher erläutert. In dieser zeigen Fig. 1 ein erstes Ausführungsbeispiel der vorliegenden Erfindung, bei welchem die Eingriffsmittel der ersten und der zweiten Knochenschraube jeweils von Außengewinden gebildet sind, Fig. 2 eine erste Knochenschraube, bei welcher die Eingriffsmittel von einem in einer Längsnut liegenden Innengewinde gebildet sind, Fig. 3 das Ausführungsbeispiel von Fig. 2 vor und Fig. 4 nach dem vollständigen Eindrehen der zweiten Knochenschraube und Fig. 5 eine weitere Ausführungsform, bei welcher die Eingriffsmittel der ersten Knochenschraube in einer Ausnehmung gebildet sind, welche die ersten Knochenschraube vollständig durchsetzt.

In Fig. 1 sind mit 1 eine erste Knochenschraube und mit 2 eine zweite Knochenschraube bezeichnet. Die erste Knochenschraube 1 weist ein selbstschneidendes Gewinde auf, welches in unterschiedlichen axialen Teilbereichen 3 und 4 unterschiedliche Ganghöhen aufweist, sodass es beim Einschrauben der Knochenschraube 1 in den Knochen zu einer Kompression der Knochenfragmente kommt. Beim Einschrauben ist die Knochenschraube 1 von einem Bohrdraht 5 geführt, mit welchem die Bohrung in die zu fixierenden Knochenfragmente gesetzt wird. Nach dem Einschrauben der ersten Knochenschraube 1 kann entlang dieser die zweite Knochenschraube 2 eingeschraubt werden, wobei die zweite Knochenschraube 2 in diesem Fall aufgrund der Tatsache, dass die Gewindeganghöhe in den axialen Teilbereichen 3 und 4 unterschiedlich ist, in einem distalen Bereich 6 ohne Gewinde ausgebildet ist und erst im proximalen Bereich 7 mit einem Außengewinde in das Gewinde der ersten Knochenschraube 1 eingreift. Wenn die beiden Knochenschrauben des Knochenschraubensets gemäß der vorliegenden Erfindung auf diese Weise eingesetzt werden, ergibt sich, dass der Gesamtquerschnitt quer zur Längsachse der beiden Schrauben nicht rotationssymmetrisch ist, und somit eine Verdrehung der fixierten Knochenfragmente um die Längsachse der Schrauben verhindert wird.

In Fig. 2 ist wiederum eine erste Knochenschraube 1 dargestellt, wobei die erste Knochenschraube 1 in diesem Fall eine Längsnut 8 trägt, in welche ein Innengewinde eingedreht ist. Das Außengewinde der Knochenschraube 1 ist wiederum als Gewinde mit unterschiedlichen Ganghöhen und somit als Kompressionsgewinde ausgebildet, wohingegen das Innengewinde in der Längsnut 8 mit konstanter Ganghöhe ausgebildet ist. Nach dem Setzen der ersten Knochenschraube 1 kann eine zweite Knochenschraube 2 unter Umständen auch ohne das Setzen einer Hilfsbohrung entlang der Längsnut 8 in das Gewinde der Längsnut 8 eingesetzt werden und entlang der Knochenschraube 1 ebenfalls in den Knochen gebohrt werden. Es ist offensichtlich, dass nach dem Einsetzen der zweiten Knochenschraube 2 in die Längsnut 8 ein Teil des Querschnitts der zweiten Knochenschraube 2 aus der gedachten kreisförmigen Hüllkurve der ersten Knochenschraube 1 heraustritt, sodass sich insgesamt ein Querschnitt ergibt, der nicht rotationssymmetrisch ist, wodurch die Verdrehung der Knochenfragmente zueinander wiederum verhindert wird. Dieser Effekt wird durch eine zusätzliche Kompression des umliegenden Knochengewebes verstärkt, indem sich die Längsachse der zweiten Knochenschraube durch das Eindrehen derselben von der Längsachse der ersten Knochenschraube entfernt. Dies wird dadurch erreicht, dass, wie in Fig. 4 ersichtlich ist, die Profilhöhe des durchgehenden Innengewindes in der Längsnut der ersten Knochenschraube im Bereich der Schraubenspitze zunimmt und der Kerndurchmesser der zweiten Knochenschraube an deren Wurzel stetig zunimmt. In Fig. 4 ist ersichtlich, dass im eingesetzten Zustand durch das Zunehmen der Gewindeprofilhöhe der ersten Schraube im distalen Bereich und durch die Zunahme des Kerndurchmessers der zweiten Schraube im proximalen Bereich eine Entfernung der Längsachsen der beiden Schrauben in der Endphase des Einsetzens der zweiten Schraube erfolgt.

In Fig. 5 ist eine besondere nicht erfindungsgemässe Ausführungsform dargestellt, bei welcher die Knochenschraube 1 voneinander getrennte axiale Teilbereiche 3 und 4 mit Gewinden unterschiedlicher Ganghöhe aufweist. Die erste Knochenschraube 1 wirkt somit wieder als Kompressionsschraube, wobei in einem mittleren Teilbereich 9 eine Durchbrechung für die Aufnahme der zweiten Knochenschraube 2 vorgesehen ist, die in einem spitzen Winkel durch die Knochenschraube 1 verläuft. Der Winkel der Durchbrechung relativ zur Längsachse der ersten Knochenschraube 1 ist hierbei so gewählt, dass die Mündung der Durchbrechung möglichst nahe an der Wurzel 9 der ersten Knochenschraube 1 mündet, sodass das Setzen der zweiten Knochenschraube 2 von der selben Seite erfolgen kann, von der die erste Knochenschraube 1 gesetzt wurde. Da die Knochenschraube 1 vollständig in Knochen und Weichteil versenkt wird, wird das Setzen der Knochenschraube 2 durch die Verwendung eines Zielgerätes erleichtert, welches beispielsweise am Schraubenzieher für die Knochenschraube 1 befestigt oder aufsetzbar sein kann.

## Patentansprüche

1. Knochenschraubenset zur Fixierung von zwei Knochenfragmenten, insbesondere zur Behandlung einer Kahnbeinfraktur oder einer Kahnbeinpseudoarthrose, umfassend eine erste Knochenschraube (1) und zumindest eine zweite Knochenschraube (2), wobei die erste Knochenschraube (1) als Kompressionsschraube ausgebildet ist, wobei die Gewindesteigung des Aussengewindes der ersten Knochenschraube (1) zur Erzielung einer Kompression in einem der Schraubenwurzel benachbarten Bereich kleiner gewählt ist als in einem der Schraubenspitze benachbarten Bereich, und die erste und die zweite Knochenschraube (1,2) miteinander zusammenwirkende Eingriffsmittel aufweisen, um die zweite Knochenschraube (2) in einer mit wenigstens einem Teilbereich derselben aus dem Querschnitt der ersten Knochenschraube (1) heraustretenden Position relativ zur ersten Knochenschraube (1) zu fixieren und wobei die erste Knochenschraube (1) an ihrem Aussenumfang eine Längsnut (8) aufweist, die zumindest über einen Teil ihrer Länge mit einem Innengewinde versehen ist.

2. Knochenschraubenset nach Anspruch 1, **dadurch gekennzeichnet, dass** die miteinander zusammenwirkenden Eingriffsmittel von einem Gewinde der zweiten Knochenschraube (2) und einem mit diesem in Eingriff bringbaren Gewinde der ersten Knochenschraube (1) gebildet sind.

3. Knochenschraubenset nach Anspruch 1 oder 2 , **dadurch gekennzeichnet, dass** die erste und die zweite Knochenschraube (1,2) in einer im Wesentlichen parallel zueinander angeordneten Position zueinander fixiert sind.

4. Knochenschraubenset nach einem der Ansprüche 1, 2, oder 3, **dadurch gekennzeichnet, dass** die Tiefe der Längsnut (8) durch Ansteigen der Gewindeprofilhöhe in Richtung zur Spitze der ersten Knochenschraube (1) abnimmt und der Kerndurchmesser der zweiten Knochenschraube (2) zur Wurzel hin zunimmt.

5. Knochenschraubenset nach einem der Ansprüche 1 bis 4, **dadurch gekennzeichnet, dass** die Längsnut (8) im Bereich des der Schraubenspitze der ersten Knochenschraube (1) benachbarten Gewindes ausläuft.

6. Knochenschraubenset nach einem der Ansprüche 1 bis 5, **dadurch gekennzeichnet, dass** die Längsnut (8) von der Wurzel der ersten Knochenschraube (1) zur Spitze der ersten Knochenschraube (1) hin aus dem Querschnitt der ersten Knochenschraube (1) austritt.

7. Knochenschraubenset nach einem der Ansprüche 1 bis 6, **dadurch gekennzeichnet, dass** die erste Knochenschraube (1) eine Durchbrechung für die Aufnahme der zweiten Knochenschraube (2) aufweist, deren Achse einen spitzen Winkel, insbesondere 10 bis 40°, mit der Schraubenachse der ersten Knochenschraube (1) einschliesst.

8. Knochenschraubenset nach Anspruch 7, **dadurch gekennzeichnet, dass** die Durchbrechung ein Innengewinde für das Einschrauben der zweiten Knochenschraube (2) aufweist.

9. Knochenschraubenset nach einem der Ansprüche 1 bis 8, **dadurch gekennzeichnet, dass** der Durchmesser der ersten Knochenschraube (1) grösser ist als der Durchmesser der zweiten Knochenschraube (2), vorzugsweise das 1,5 - 2,5-fache des Durchmessers der zweiten Knochenschraube (2) beträgt.

10. Knochenschraubenset nach einem der Ansprüche 1 bis 9, **dadurch gekennzeichnet, dass** die erste Knochenschraube (1) konisch ausgebildet ist.

11. Knochenschraubenset nach einem der Ansprüche 1 bis 10, **dadurch gekennzeichnet, dass** die erste und die zweite Knochenschraube (1,2) ein selbstschneidendes Aussengewinde aufweisen.

12. Knochenschraubenset nach einem der Ansprüche 1 bis 11, **dadurch gekennzeichnet, dass** die zweite Knochenschraube (2) einen gewindelosen Bereich im Bereich der Schraubenwurzel aufweist.

13. Knochenschraubenset nach einem der Ansprüche 1 bis 12, **dadurch gekennzeichnet, dass** die erste Knochenschraube (1) geschlitzt ausgeführt ist und ausdrückbare verdrehschützende Elemente aufweist.

14. Knochenschraubenset nach einem der Ansprüche 1 bis 13 umfassend drei oder mehr Knochenschrauben, **dadurch gekennzeichnet, dass** die erste und weitere Knochenschrauben (1,2) miteinander zusammenwirkende Eingriffsmittel aufweisen, um die weiteren Knochenschrauben (2) in einer mit wenigstens einem Teilbereich der selben aus dem Querschnitt der ersten Knochenschraube (1) heraustretenden Position relativ zur ersten Knochenschraube (1) zu fixieren.

## Claims

1. Bone screw set for fixing two bone fragments, particularly for treating a scaphoid bone fracture or a scaphoid bone pseudarthrosis, comprising a first bone screw (1) and at least a second bone screw (2), wherein the first bone screw (1) is configured as a compression screw, wherein the thread lead of the outer thread of the first bone screw (1) is selected to be smaller in a region adjacent to the root of the screw than in a region adjacent to the tip of the screw to achieve a compression, and the first and the second bone screws (1, 2) have engagement means interacting with respect to one another in order to fix the second bone screw (2) in a position, relative to the first bone screw (1), protruding with at least a partial portion thereof out of the cross section of the first bone screw (1), and wherein the first bone screw (1) has a longitudinal groove (8) on its outer circumference, which is provided with an internal thread at least over a part of its length.

2. Bone screw set according to claim 1, **characterized in that** the engagement means interacting with respect to one another are formed by a thread of the second bone screw (2) and a thread of the first bone screw (1) that can engage it.

3. Bone screw set according to claim 1 or 2, **characterized in that** the first and the second bone screws (1, 2) are fixed with respect to one another in a position essentially parallel with respect to one another.

4. Bone screw set according to any one of claims 1, 2, or 3, **characterized in that** the depth of the longitudinal groove (8) decreases due to the increasing of the thread profile height in a direction towards the tip of the first bone screw (1) and the core diameter of the second bone screw (2) increases towards the root.

5. Bone screw set according to any one of claims 1 to 4, **characterized in that** the longitudinal groove (8) shallows out in the region of the thread adjacent to the tip of the screw of the first bone screw (1).

6. Bone screw set according to any one of claims 1 to 5, **characterized in that** the longitudinal groove (8) protrudes, from the root of the first bone screw (1) to the tip of the first bone screw (1), from the cross section of the first bone screw (1).

7. Bone screw set according to any one of claims 1 to 6, **characterized in that** the first bone screw (1) has an opening for receiving the second bone screw (2), whose axis forms an acute angle, particularly of 10 to 40°, with the screw axis of the fist bone screw (1).

8. Bone screw set according to claim 7, **characterized in that** the opening has an internal thread for screwing the second bone screw (2).

9. Bone screw set according to any one of claims 1 to 8, **characterized in that** the diameter of the first bone screw (1) is larger than the diameter of the second bone screw (2), preferably 1.5 to 2.5 times the diameter of the second bone screw (2).

10. Bone screw set according to any one of claims 1 to 9, **characterized in that** the first bone screw (1) is configured in a conical way.

11. Bone screw set according to any one of claims 1 to 10, **characterized in that** the first and the second bone screws (1, 2) have a self-cutting external thread.

12. Bone screw set according to any one of claims 1 to 11, **characterized in that** the second bone screw (2) has an unthreaded region in the region of the root of the screw.

13. Bone screw set according to any one of claims 1 to 12, **characterized in that** the first bone screw (1) is carried out in a slotted way and has anti-twist elements which can be pushed out.

14. Bone screw set according to any one of claims 1 to 13, comprising three or more bone screws, **characterized in that** the first and further bone screws (1, 2) have engagement means interacting with respect to one another in order to fix the further bone screws (2) in a position, relative to the first bone screw (1), protruding with at least a partial portion thereof out of the cross section of the first bone screw (1).

## Revendications

1. Ensemble de vis pour ostéosynthèse pour la fixation de deux fragments d'os, en particulier, pour le traitement d'une fracture de l'os scaphoïde ou d'une pseudarthrose du scaphoïde, comprenant une première vis pour ostéosynthèse (1) et, au moins, une deuxième vis pour ostéosynthèse (2), dans lequel la première vis pour ostéosynthèse (1) est configurée comme une vis de compression, dans laquelle le pas de filet du filet extérieur de la première vis pour ostéosynthèse (1) est choisi pour être plus petit dans une région adjacente à la racine de la vis que dans une région adjacente à la pointe de la vis pour obtenir une compression et la première et la deuxième vis pour ostéosynthèse (1,2) présentent des moyens d'engagement coopérant les uns par rapport aux autres afin de fixer la deuxième vis pour ostéosynthèse (2) dans une position, par rapport à la première vis pour ostéosynthèse (1) avec, au moins, une partie de cette dernière en saillie à l'extérieur de la section transversale de la première vis pour ostéosynthèse (1), et dans lequel la première vis pour ostéosynthèse (1) présente une rainure longitudinale (8) sur sa circonférence extérieure munie d'un filet intérieur, au moins, sur une partie de sa longueur.

2. Ensemble de vis pour ostéosynthèse selon la revendication 1, **caractérisé en ce que** les moyens d'engagement qui interagissent les uns par rapport aux autres sont formés par un filet de la deuxième vis pour ostéosynthèse (2) et un filet de la première vis pour ostéosynthèse (1) pouvant être engagé avec ce dernier.

3. Ensemble de vis pour ostéosynthèse, selon la revendication 1 ou 2, **caractérisé en ce que** la première et la deuxième vis pour ostéosynthèse (1, 2) sont fixées l'une par rapport à l'autre dans une position essentiellement parallèle l'une à l'autre.

4. Ensemble de vis pour ostéosynthèse, selon l'une quelconque des revendications 1, 2 ou 3, **caractérisé en ce que** la profondeur de la rainure longitudinale (8) diminue en raison de l'augmentation de la hauteur du profil de filet dans une direction vers l'extrémité de la première vis pour ostéosynthèse (1) et le diamètre de noyau de la deuxième vis pour ostéosynthèse (2) augmente vers la racine.

5. Ensemble de vis pour ostéosynthèse, selon l'une quelconque des revendications 1 à 4, **caractérisé en ce que** la rainure longitudinale (8) saillit de la région du filet adjacente à la pointe de la vis de la première vis pour ostéosynthèse (1).

6. Ensemble de vis pour ostéosynthèse, selon l'une quelconque des revendications 1 à 5, **caractérisé en ce que** la rainure longitudinale (8) saillit de la racine de la première vis pour ostéosynthèse (1) jusqu'à la pointe de la vis de la première vis pour ostéosynthèse (1), de la section transersale de la première vis pour ostéosynthèse (1).

7. Ensemble de vis pour ostéosynthèse, selon l'une quelconque des revendications 1 à 6, **caractérisé en ce que** la première vis pour ostéosynthèse (1) présente une ouverture pour recevoir la deuxième vis pour ostéosynthèse (2), dont l'axe forme un angle aigu, en particulier, de 10 à 40°, avec l'axe de vis de la première vis pour ostéosynthèse (1).

8. Ensemble de vis pour ostéosynthèse, selon la revendication 7, **caractérisé en ce que** l'ouverture présente un filet intérieur pour le vissage de la deuxième vis pour ostéosynthèse (2).

9. Ensemble de vis pour ostéosynthèse, selon l'une quelconque des revendications 1 à 8, **caractérisé en ce que** le diamètre de la première vis pour ostéosynthèse (1) est plus grand que le diamètre de la deuxième vis pour ostéosynthèse (2), préférablement 1,5 à 2,5 fois le diamètre de la deuxième vis pour ostéosynthèse (2).

10. Ensemble de vis pour ostéosynthèse, selon l'une quelconque des revendications 1 à 9, **caractérisé en ce que** la première vis pour ostéosynthèse (1) est configurée de façon conique.

11. Ensemble de vis pour ostéosynthèse, selon l'une quelconque des revendications 1 à 10, **caractérisé en ce que** la première et la deuxième vis pour ostéosynthèse (1, 2) présentent un filet extérieur tranchant.

12. Ensemble de vis pour ostéosynthèse, selon l'une quelconque des revendications 1 à 11, **caractérisé en ce que** la deuxième vis pour ostéosynthèse (2) présente une région non filetée dans la région de la racine de la vis.

13. Ensemble de vis pour ostéosynthèse, selon l'une quelconque des revendications 1 à 12, **caractérisé en ce que** la première vis pour ostéosynthèse (1) est réalisée comme étant fendue et comporte des éléments anti-torsion qui peuvent être repoussés.

14. Ensemble de vis pour ostéosynthèse, selon l'une quelconque des revendications 1 à 13, comprenant trois ou plusieurs vis pour ostéosynthèse, **caractérisé en ce que** la première et les vis pour ostéosynthèse supplémentaires (1, 2) présentent des moyens d'engagement coopérant les uns par rapport aux autres afin de fixer les vis pour ostéosynthèse supplémentaires (2) dans une position, par rapport à la première vis pour ostéosynthèse (1) avec, au moins, une partie en saillie de cette dernière à l'extérieur de la section transversale de la première vis pour ostéosynthèse (1).
